(19) Europäisches Patentamt
European Patent Office
Office européen des brevets

(11) Publication number: **0 148 272 B1**

(12) **EUROPEAN PATENT SPECIFICATION**

(45) Date of publication of patent specification: **18.09.91**

(51) Int. Cl.5: **C12P 13/00**, //C12P7/02, (C12P13/00,C12R1:06), (C12P13/00,C12R1:38), (C12P13/00,C12R1:66), (C12P13/00,C12R1:785), (C12P13/00,C12R1:05)

(21) Application number: **84901806.4**

(22) Date of filing: **01.05.84**

(86) International application number: **PCT/JP84/00225**

(87) International publication number: **WO 84/04543 (22.11.84 84/27)**

# (54) PROCESS FOR PRODUCING OPTICALLY ACTIVE BENZYL ALCOHOL COMPOUNDS.

(30) Priority: **09.05.83 JP 81442/83**

(43) Date of publication of application: **17.07.85 Bulletin 85/29**

(45) Publication of the grant of the patent: **18.09.91 Bulletin 91/38**

(84) Designated Contracting States: **CH DE FR GB LI NL**

(56) References cited:
EP-A- 0 036 774
FR-A- 2 295 933
GB-A- 2 097 393
JP-A-57 115 184
US-A- 3 607 651
FR-A- 2 053 218
FR-A- 2 447 899
JP-A-56 156 90
JP-B- 5 615 233

(73) Proprietor: **SUMITOMO CHEMICAL COMPANY, LIMITED**
**Kitahama 4-chome 5-33**
**Chuo-ku Osaka 541(JP)**

(72) Inventor: **MITSUDA, Satoshi 10-439, Sonehigashimachi 2-chome**
**Toyonaka-shi**
**Osaka 561(JP)**
Inventor: **MATSUO, Noritada 29-2, Minamino-Aza-Yamamichi**
**Itami-shi**
**Hyogo 664(JP)**
Inventor: **HIROHARA, Hideo 31-18, Takakuradai 4-cho**
**Sakai-shi**
**Osaka 590-01(JP)**

EP 0 148 272 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

AGRICULTURAL & BIOLOGICAL CHEMISTRY, vol. 45, no. 6, June 1981, pages 1389-1392, Tokyo, JP; S. IRIUCHIJIMA et al.: "Asymmetric hydrolysis of (+/-)-alpha-substituted carboxylic acid esters with microorganisms"

PATENTS ABSTRACTS OF JAPAN, vol. 5, no. 111 (C-63)[783], 18th July 1981; & JP-A-56 48 888 (SAGAMI CHUO KAGAKU KENKYUSHO) 02-05-1981

J.Agric.Chem.Soc.Jaoan, vol. 50, no.10, pp.31-37, 1976

Agr.Biol.Chem., 38(10), pp. 1961-1964, 1974

"Enzymes in Organic Synthesis, CIBA Foundation Symposium III, pp. 128-129, 1985

"Rodds Chemistry of Carbon Compounds", Ed. S. Coffey, pp. 186-187, Elsevier 1968

Agric.Biol.Chem., 50(12), pp. 3181-3184, 1986

Pestic.Sci., 1980, 11, pp. 188-201

(74) Representative: Myerscough, Philip Boyd et al
J.A.Kemp & Co. 14, South Square Gray's Inn
London, WC1R 5EU(GB)

## Description

The present invention relates to the production of an optically active benzyl alcohol compound.

It is known, as a general proposition, that ester groups attached to asymmetric carbon atoms may be hydrolysed by esterases originating from micro-organisms. Agric.Biol.Chem., 45(6), 1389-1392, 1981, for example, describes experiments involving the stereoselective hydrolysis of methyl 2-phenylpropionate using strains of bacteria and of Aspergilli.

The present invention now provides a process for producing an (S)-isomer-rich optically active α-cyano-benzyl alcohol compound represented by the formula (II):

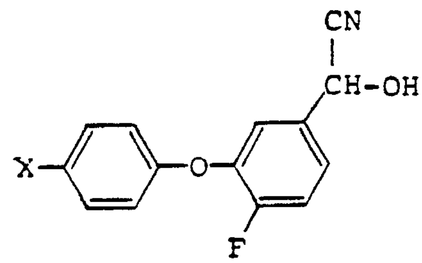

(II)

wherein X is a hydrogen atom, fluorine atom, chlorine atom or bromine atom,

which comprises allowing an esterase originating from a microorganism belonging to a genus selected from Arthrobacter, Alcaligenes, Achromabacter, Pseudomonas, Chromobacterium, Bacillus, Nocardia, Rhodotorula, Candida, Rhizopus, Mucor, Aspergillus, Hansenula and Torulopsis to react at a pH of 7 or less with an ester of the (R,S)-isomer of an α-cyano-benzyl alcohol compound, the said ester being represented by the formula (I):

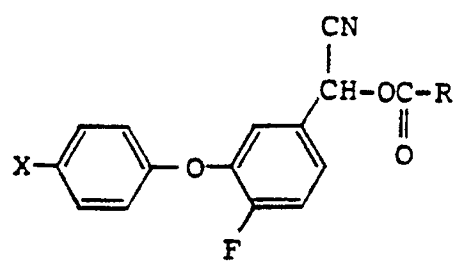

(I)

wherein X has the same meaning as above; R means a hydrogen atom, a $C_1$-$C_{18}$ alkyl, $C_2$-$C_{18}$ alkenyl, $C_2$-$C_{18}$ alkynyl, $C_1$-$C_4$ halogen-substituted alkyl, $C_2$-$C_4$ halogen-substituted alkenyl, $C_2$-$C_4$ halogen-substituted alkynyl, $C_1$-$C_8$ alkoxyl, $C_2$-$C_8$ alkenyloxy or $C_2$-$C_8$ alkynyloxy group, in order to hydrolyze the ester asymmetrically until it is resolved into an (S)-isomer-rich α-cyano-benzyl alcohol compound represented by the formula (II) and an antipode ester thereof.

The α-cyano-benzyl alcohol compound represented by the formula (II) mentioned above has been known as novel alcoholic moiety of a series of ester compounds of synthetic pyrethroids which have been excellent in insecticidal activity.

Since the α-cyano-benzyl alcohol compound has an asymmetric carbon at the α-position there are two optical isomers. With respect to insecticidal activity as an ester, the (S)-isomer ester of α-cyano-benzyl alcohol compound is much superior to the antipode (R)-isomer ester. Accordingly, it has been desired to develop commercially advantageous technology to resolve optically the (R,S)-isomer of said α-cyano-benzyl alcohol compound into the (S)-isomer thereof. However, since said α-cyano-benzyl compound is unstable, the optical resolution thereof is not easy. The current situation is that the method now available for the optical resolution of said alcohol requires complicated processes and expensive optically active agents.

The present inventors have been investigating to find out a commercially advantageous process for producing (S)-isomer of α-cyano-benzyl alcohol compound represented by the formula (II) mentioned above. At last, they have found that the biochemically asymmetric hydrolysis of a racemic isomer, namely, an (R,S)-isomer of the ester of the α-cyano-benzyl alcohol compound represented by the formula (I), which

is the starting material, efficiently resolves the ester into (S)-isomer-rich optically active $\alpha$-cyano-benzyl alcohol compound represented by the formula (II) and the antipode ester thereof. They have accomplished the present invention, adding various further investigations to the finding above.

That is, the present invention a process for producing an (S)-isomer-rich optically active $\alpha$-cyano-benzyl alcohol compound represented by the formula (II) mentioned above by allowing an esterase (in the present invention, esterase means esterase in a broad sense including lipase) originated from a microorganism selected from the genera listed above to react at not higher than pH 7 with an ester of the (R,S)-$\alpha$-cyano-benzyl alcohol represented by the formula (I) mentioned above in order to asymmetrically hydrolyze the same until it is resolved into an (S)-isomer rich $\alpha$-cyano-benzyl alcohol represented by the formula (II) and an antipode ester thereof.

The more detailed description of the process of the present invention will be given hereinafter.

The microorganisms which produce the esterase used in the present invention are selected from the genera Arthrobacter, Alcaligenes, Achromobacter, Pseudomonas, Chromobacterium, Bacillus, Nocardia, Rhodotorula, Candida, Rhizopus, Mucor, Aspergillus, Hansenula and Torulopsis. Amongst these, from the viewpoints of hydrolysis capability and optical purity of the optically active $\alpha$-cyano-benzyl alcohol compound represented by the formula (II), the esterase produced by microorganisms belonging to genuses Arthrobacter, Alcaligenes, Achromobacter, Pseudomonas and Chromobacterium is preferable.

As the typical examples of microorganisms belonging to those genera mentioned above, there can be illustrated following strains:

| | | |
|---|---|---|
| (1) | Arthrobacter simplex | IFO-3530 |
| (2) | Alcaligenes faecalis | IFO-12669 |
| (3) | Achromobacter Sp. | ATCC-21910 |
| (4) | Pseudomonas fluorescens | IFO-3081 |
| (5) | Chromobacterium viscosum | ATCC-6918 |
| (6) | Bacillus licheniformis | IFO-12197 |
| (7) | Nocardia erthyropolis | IFO-12320 |
| (8) | Rhodotorula minuta var. texensis | IFO-0879 |
| (9) | Candida utilis | IFO-1086 |
| (10) | Rhizopus chinensis | IFO-4737 |
| (11) | Mucor javanicus | IFO-4572 |
| (12) | Aspergillus var. asper | IFO-5324 |
| (13) | Hansenula anomala | IFO-0707 |
| (14) | Torulopsis candida | IFO-0380 |

All these strains have been deposited at Institute of Fermentation, Osaka (IFO) or American Type Culture Collection (ATCC), and are available from these depositories.

Some of the esterase originated from microorganisms have been commercially available in the market. Also these enzymes commercially availble in the market can be used for the present invention.

The enzymes commercially available in the market which can be used for the present invention are illustrated below.

| Name of enzyme | Origin | Selling or manufacturing company |
|---|---|---|
| Lipase AP | genus Aspergillus | Amano Seiyaku |
| Lipase M-AP | genus Mucor | " |
| Lipase "Amano" P | genus Pseudomonas | " |
| Lipase "Godo" BSL | genus Arthrobacter | Godo Shusei |
| Lipase | " | Shinnihon Kagaku |
| Lipase "Toyo" | genus Chromobacterium | Toyo Jozo |
| Lipase "Saiken" | genus Rhizopus | Osaka Saikin Kenkyusho |
| Lipase AL | genus Achromobacter | Meito Sangyo |
| Lipase PL266 | genus Alcaligenes | " |
| Lipase PL679 | " | " |

In carrying out the present invention, the asymmetric hydrolysis of the (R,S)-isomer of the ester represented by the formula (I) mentioned above is conducted by mixing cultured liquor of such microorganisms, filtrate of the cultured liquor, suspension of microorganism cells or aqueous solution containing treated product thereof such as crude esterase or purified esterase and said (R,S)-isomer of the ester, and stirring or shaking the mixture. If necessary, a nonester type surfactant may be added. Also it is possible for the enzyme to be in the immobilized form.

The suitable reaction temperature in this reaction is 10 to 65° C, preferably 20 to 50° C. The reaction time usually ranges from 3 to 48 hours. However, the reaction time can be shortened by elevating the reaction temperature or using enzymes having higher activities.

It is important to keep the pH in the reaction at not higher than pH 7, preferably in a range of pH 3.5 to pH 6.0.

Furthermore, it is preferable to keep the pH constant by utilization of a buffer solution, in order to prevent pH from lowering due to an organic acid such as acetic acid by-produced from the hydrolysis. As the buffer solution, either one containing inorganic acid salt or organic acid salt may be used.

The substrate,the (R,S)-isomer of the ester represented by the formula (I) may be used in a concentration of 1 to 80 w/w %, preferably 5 to 40 w/w % on the basis of the reaction liquid.

After carrying out the asymmetric hydrolysis reaction as mentioned above, the optically active α-cyano-benzyl alcohol compound represented by the formula (II) in the free form is separated from the unreacted ester of the antipode alcohol compound and they are recovered by an operation such as liquid phase separation by standing, extraction with a solvent or column chromatography.

Such a separation and recovery operation is conducted, for example, in such a manner that the reaction mixture is extracted with an organic solvent such as ether, benzene or toluene, and the extract is subjected to column chromatography using silica gel, etc. and a cyclohexaneether (95:5) solution as an eluent to isolate the free optically active α-cyano-benzyl alcohol compound represented by the formula (II) and the unreacted antipode ester, respectively.

In addition, the unreacted organic carboxylic acid ester which has been separated and recovered as mentioned above is subjected to racemization by bringing it in contact with a base compound such as ammonia, pyridine or triethylamine, and can be used again as the starting material for the process of the

present invention.

The (R,S)-isomer of the ester represented by the formula (I) which is used as the starting material in the present invention can be easily obtained by an ordinary process for producing ester, for instance, by a process wherein a halide of an organic carboxylic acid represented by the formula (III):

$$R_1-\underset{\underset{O}{\|}}{C}-OH \qquad (III)$$

wherein $R_1$ is a hydrogen atom, a $C_1$-$C_{18}$ alkyl, $C_2$-$C_{18}$ alkenyl, $C_2$-$C_{18}$ alkynyl, $C_1$-$C_4$ halogen-substituted alkyl, $C_2$-$C_4$ halogen-substituted alkenyl or $C_2$-$C_4$ halogen-substituted alkynyl group, for example, acid chloride or acid bromide, or an acid anhydride thereof is allowed to react with an (R,S)-isomer of α-cyano-benzyl alcohol compound represented by the formula (II), or by a process wherein a halide of the organic carboxylic acid represented by the formula (III) mentioned above, for example, acid chloride or acid bromide, an aldehyde compound represented by the formula (IV):

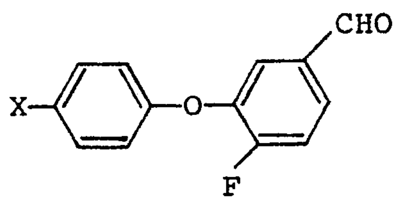

(IV)

wherein X has the same meaning as above, and sodium cyanide are allowed to react, or by a process wherein one of chloroformic acid esters represented by the formula (V):

$$R_2-O-\underset{\underset{O}{\|}}{C}-Cl \qquad (V)$$

wherein $R_2$ is a $C_1$-$C_8$ alkyl, $C_2$-$C_8$ alkenyl or
$C_2$-$C_8$ alkynyl group,
is allowed to react with an (R,S)-isomer of α-cyano-benzyl alcohol compound represented by the formula (II).

As the organic carboxylic acid represented by the formula (III) mentioned above, there are illustrated, for example, formic acid, acetic acid, propionic acid, butyric acid, valeric acid, isovaleric acid, caproic acid, caprylic acid, lauric acid, myristic acid, palmitic acid, stearic acid, oleic acid, linoleic acid, or halogen-substituted compounds thereof and the like. On account of the easiness in handling and the optical purity of the reaction product obtained by the asymmetric hydrolysis, a $C_2$-$C_{12}$ fatty acid or a $C_1$-$C_4$ halogen-substituted fatty acid having one chlorine or bromine atom at the α-position thereof is preferable; further, from the viewpoints of easiness in availability and economy, acetic acid, propionic acid, monochloroacetic acid or monobromoacetic acid is more preferable.

Also as chloroformic acid ester represented by the formula (V), methyl chlorocarbonate, ethyl chlorocarbonate, propyl chlorocarbonate and the like are preferable from the viewpoints of easiness in availability and economy.

Furthermore, the process of the present invention can be applied to the case wherein the substrate is a half-ester of an organic carboxylic acid of α-cyano-benzyl alcohol compound represented by the formula (II) in place of the ester represented by the formula (I).

**The following Examples further illustrate the invention:**

Examples 1-8

To 15 mℓ each of an acetate buffer solution having a concentration of 0.2 M (pH 4.0) were added 1.0 g each of acetic acid ester of (R,S)-α-cyano-3-phenoxy-4-fluorobenzyl alcohol and 40 mg each of esterases described in Table 1. The mixtures were allowed to react at 40 °C with stirring. After the reaction was conducted for 24 hours, the reaction mixture each was extracted with toluene. The extract was analyzed with a high-performance liquid chromatography (HPLC) [Lichrosorb RP-18, methanol-water (6:4), 254nm, UV detection], and conversion rate was calculated from the peak area ratio of α-cyano-3-phenoxy-4-fluorobenzyl alcohol with acetic acid ester of α-cyano-3-phenoxy-4-fluorobenzyl alcohol.

After concentrating the extract each, the concentrate was subjected to a silica gel column chromatography. The elution was conducted with a solution of cyclohexaneethyl ether (95:5) to separate and remove unaltered acetic acid ester of α-cyano-3-phenoxy-4-fluorobenzyl alcohol. Then, the elution was additionally conducted with methanol containing a small amount ($10^{-5}$%) of p-toluenesulfonic acid until the free α-cyano-3-phenoxy-4-fluorobenzyl alcohol was obtained.

In 1 mℓ each of toluene was dissolved 10 mg each of the free α-cyano-3-phenoxy-4-fluorobenzyl alcohol thus obtained. To this solution each was added equimolar chloride of (S)-(+)-2-(4-chlorophenyl)-isovaleric acid together with pyridine. The mixtures were allowed to react to produce (S)-(+)-2-(4-chlorophenyl)-isovaleric diastereomer of α-cyano-3-phenoxy-4-fluorobenzyl alcohol, respectively. The analysis of the optical isomers was conducted with gas chromatography (column: DCQF-1, 2.5 m, column temperature: 250 °C).

The results are shown in Table 1.

Table 1

| Example No. | Origin of esterase | Conversion rate (%) | Ratio of optical isomers of free α-cyano-3-phenoxy-4-fluorobenzyl alcohol [(S)-isomer : (R)-isomer] |
|---|---|---|---|
| 1 | genus Arthrobacter (Lipase Godo BSL) | 49.6 | 95.8 : 4.2 |
| 2 | genus Chromobacterium (Lipase "Toyo") | 50.0 | 95.3 : 4.7 |
| 3 | genus Pseudomonas (Lipase "Amano") | 47.9 | 97.5 : 2.5 |
| 4 | genus Aspergillus (Lipase AP) | 44.2 | 79.6 : 20.4 |
| 5 | genus Mucor (Lipase M-AP) | 9.4 | 73.6 : 26.4 |
| 6 | genus Alcaligenes (Lipase PL-679) | 46.2 | 98.1 : 1.9 |
| 7 | genus Achromobacter (Lipase AL) | 47.2 | 93.8 : 6.2 |
| 8 | genus Rhizopus (Lipase "Saiken") | 13.4 | 80.9 : 19.1 |

Examples 9-13

To 15 mℓ each of 0.2M concentration of an acetate buffer solution (pH 4.0) were added 2.0 g each of various esters of (R,S)-isomer of α-cyano-benzyl alcohol mentioned in Table 2, the substrate and 40 mg

each of esterases listed in Table 2. The mixtures were allowed to react at 40 °C with stirring. Thereafter the same operation as in Examples 1-8 was conducted. The results are shown in Table 2.

Table 2

| Example No. | Origin of esterase | Substrate | Conversion rate (%) | Ratio of optical isomers of free α-cyano-3-phenoxybenzyl alcohol compounds [(S)-isomer : (R)-isomer] |
|---|---|---|---|---|
| 9 | genus Arthrobacter (Lipase GODO BSL) | Monochloroacetic acid ester of (R,S)-α-cyano-3-(4-chlorophenoxy)-4-fluorobenzyl alcohol | 50.2 | 96.8 : 3.2 |
| 10 | genus Arthrobacter (Lipase GODO BSL) | Acetic acid ester of (R,S)-α-cyano-3-(4-fluorophenoxy)-4-fluorobenzyl alcohol | 48.9 | 99.3 : 0.7 |
| 11 | genus Arthrobacter (Lipase GODO BSL) | Acetic acid ester of (R,S)-α-cyano-3-(4-bromophenoxy)-4-fluorobenzyl alcohol | 49.0 | 99.5 : 0.5 |
| 12 | genus Pseudomonas (Lipase "Amano" P) | Ethyl carbonic acid ester of (R,S)-α-cyano-3-(4-chlorophenoxy)-4-fluorobenzyl alcohol | 47.8 | 97.7 : 2.3 |

Table 2 (cont'd)

| Example No. | Origin of esterase | Substrate | Conversion rate (%) | Ratio of optical isomers of free α-cyano-3-phenoxy-benzyl alcohol compounds [(S)-isomer : (R)-isomer] |
|---|---|---|---|---|
| 13 | genus Chromobacterium (Lipase "Toyo") | Ethyl carbonic acid ester of (R,S)-α-cyano-3-(4-fluoro-phenoxy)-4-fluoro-benzyl alcohol | 43.0 | 96.6 : 3.4 |
| 14 | genus Arthrobacter (Lipase: Shinnihon Kagaku) | Monochloroacetic acid ester of (R,S)-α-cyano-3-phenoxy-4-fluorobenzyl alcohol | 50.6 | 98.2 : 1.8 |
| 15 | genus Pseudomonas (Lipase "Amano" P) | Monobromoacetic acid ester of (R,S)-α-cyano-3-phenoxy-4-fluorobenzyl alcohol | 51.5 | 96.5 : 3.5 |
| 16 | genus Arthrobacter (Lipase: Sinnihon Kagaku) | Ethyl carbonic acid ester of (R,S)-α-cyano-3-phenoxy-4-fluorobenzyl alcohol | 50.0 | 100 : 0 |

Examples 17-23

In flasks with shoulder (500 mℓ each) were charged 100 mℓ each of a liquid medium, respectively. The medium was sugared bouillon medium for bacteria in Examples 17-19, which is prepared by dissolving 10.0 g of glucose, 5.0 g of peptone and 5.0 g of meat extract in 1 ℓ of water and adjusting the pH to 7.2, or a malt extract-yeast extract medium for fungi and yeast in Examples 20-23, which is prepared by dissolving 5.0 g of peptone, 10.0 g of glucose, 3.0 g of malt extract and 3.0 g of yeast extract in 1 ℓ of water and adjusting the pH to pH 6.5. After sterilization, the medium each was inoculated with 2 platinum loops of

slant cultured microorganisms as described in Table 3 and cultured on a reciprocating shaker at 30 ° C for 72 hours.

Then, pH value of the cultured medium each was adjusted to pH 4.5 by using a 2M aqueous solution of hydrochloric acid. To the cultured medium each was added 3.0 g each of acetic acid ester of (R,S)-α-cyano-3-phenoxy-4-fluorobenzyl alcohol. The mixtures were allowed to react at 30 ° C for 30 hours with stirring, respectively. Thereafter, the separation of the reaction product was conducted in the same manner as in Example 1 and the ratio of optical isomers and the conversion rate of α-cyano-3-phenoxy-4-fluorobenzyl alcohol obtained were measured.

The results are shown in Table 3.

Table 3

| Example No. | Origin of esterase (cultured micro-organisms) | Conversion rate (%) | Ratio of optical isomers of free α-cyano-3-phenoxy-4-fluorobenzyl alcohol [(S)-isomer : (R)-isomer] |
|---|---|---|---|
| 17 | Nocardia erythropolis IFO-12320 | 38.3 | 86.5 : 13.5 |
| 18 | Bacillus sphaericus IFO-3528 | 21.7 | 62.4 : 37.6 |
| 19 | Arthrobacter sp. ATCC-21908 | 50.3 | 90.2 : 9.8 |
| 20 | Rhodotorula minuta var. texensis IFO-0879 | 47.5 | 82.0 : 18.0 |
| 21 | Torulopsis candida IFO-0380 | 30.6 | 73.2 : 26.8 |
| 22 | Candida utilis IFO-1086 | 35.5 | 66.7 : 33.3 |
| 23 | Hansenula anomala IFO-0707 | 41.3 | 92.1 : 7.9 |

Examples 24-29

Each of microorganisms described in Table 4 was cultured in the same process as in Examples 17-23. Then, the pH of each culture medium was adjusted to pH 4.5 by using a 2M aqueous HCℓ solution. To this medium each was added 3.0 g of acetic acid ester of (R,S)-α-cyano-3-(4-chlorophenoxy)-4-fluorobenzyl alcohol, and the mixture was allowed to react at 30° C for 30 hours with stirring. Thereafter, the separation of the reaction product was conducted in the same manner as in Example 1, and the ratio of optical isomers

and the conversion rate of α-cyano-3-(4-chlorophenoxy)-4-fluorobenzyl alcohol obtained were measured.

The results are shown in Table 4.

Table 4

| Example No. | Origin of esterase (cultured micro-organisms) | Conversion rate (%) | Ratio of optical isomers of free α-cyano-3-(4-chlorophenoxy)-4-fluorobenzyl alcohol [(S)-isomer : (R)-isomer] |
|---|---|---|---|
| 24 | Nocardia erythropolis IFO-12320 | 31.8 | 79.7 : 20.3 |
| 25 | Bacillus sphaericus IFO-3528 | 32.0 | 70.7 : 29.3 |
| 26 | Rhodotorula minuta var. texensis IFO-0879 | 47.6 | 83.9 : 16.1 |
| 27 | Torulopsis candida IFO-0380 | 39.8 | 81.0 : 19.0 |
| 28 | Candida utilis IFO-1086 | 42.3 | 76.6 : 23.4 |
| 29 | Hansenula anomala IFO-0707 | 48.3 | 69.4 : 30.6 |

## Claims

1. A process for producing an (S)-isomer-rich optically active α-cyano-benzyl alcohol compound represented by the formula (II):

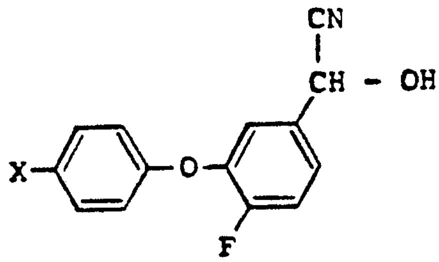

(II)

wherein X is a hydrogen atom, fluorine atom, chlorine atom or bromine atom,

which comprises allowing an esterase originating from a micro-organism belonging to a genus selected from Arthrobacter, Alcaligenes, Achromobacter, Pseudomonas, Chromobacterium, Bacillus, Nocardia, Rhodotorula, Candida, Rhizopus, Mucor, Aspergillus, Hansenula and Torulopsis to react at a pH of 7 or less with an ester of the (R,S)-isomer of the α-cyano-benzyl alcohol compound, the said ester being represented by the formula:

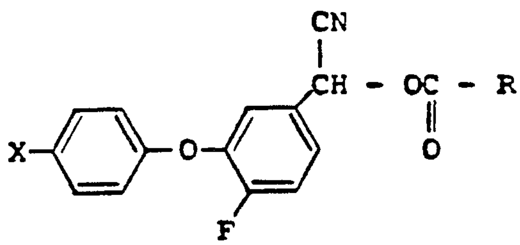

(I)

wherein X has the same meaning as above; R means a hydrogen atom, a $C_1$-$C_{18}$ alkyl, $C_2$-$C_{18}$ alkenyl, $C_2$-$C_{18}$ alkynyl, $C_1$-$C_4$ halogen-substituted alkyl, $C_2$-$C_4$ halogen-substituted alkenyl, $C_2$-$C_4$ halogen-substituted alkynyl, $C_1$-$C_8$ alkoxyl, $C_2$-$C_8$ alkenyloxy or $C_2$-$C_8$ alkynyloxy group, in order to hydrolyze the ester asymmetrically until it is resolved into an (S)-isomer rich α-cyano-benzyl alcohol compound represented by the formula (II) and an antipode ester thereof.

2. A process according to Claim 1 wherein the esterase originates from a microorganism belonging to genus Arthrobacter, genus Alcaligenes, genus Achromobacter, genus Pseudomonas or genus Chromobacterium.

3. A process according to Claim 1 or 2 wherein the substituent group R in the ester of formula (I) is a $C_1$-$C_{11}$ alkyl group, $C_2$-$C_{11}$ alkenyl group, $C_2$-$C_{11}$ alkynyl group, $C_1$-$C_4$ alkyl group having a chlorine atom or bromine atom at the α-position thereof, $C_2$-$C_4$ alkenyl group having a chlorine atom or bromine atom at the α-position thereof, $C_2$-$C_4$ alkynyl group having a chlorine atom or bromine atom at the α-position thereof, or $C_1$-$C_3$ alkoxyl group.

4. A process according to Claim 1, 2 or 3 wherein the substituent group R in the ester of formula (I) is a methyl group, ethyl group, monochloromethyl group, monobromomethyl group, methoxy group, ethoxy group or propoxy group.

5. A process according to any one of claims 1 to 4 wherein the asymmetric hydrolysis reaction is conducted at a pH of from 3.5 to 6.0.

6. A process according to any one of claims 1 to 5 wherein the asymmetric hydrolysis reaction is conducted at 20-50 °C.

## Revendications

1. Procédé de préparation d'un alcool α-cyano-benzylique optiquement actif, riche en isomère-(S), de formule (II) :

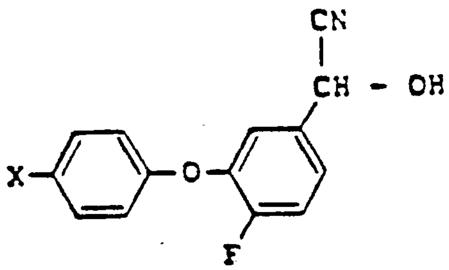

(II)

dans laquelle X est un atome d'hydrogène, un atome de fluor, un atome de chlore ou un atome de brome,
qui consiste à faire réagir une estérase provenant d'un microorganisme appartenant à un genre choisi parmi Arthrobacter, Alcaligenes, Achromobacter, Pseudomonas, Chromobacterium, Bacillus, Nocardia, Rhodotorula, Candida, Rhizopus, Mucor, Aspergillus, Hansenula et Torulopsis, à un pH de 7 ou à un pH inférieur à 7, sur un ester de l'isomère (R,S) de l'alcool α-cyano-benzylique, cet ester étant représenté par la formule :

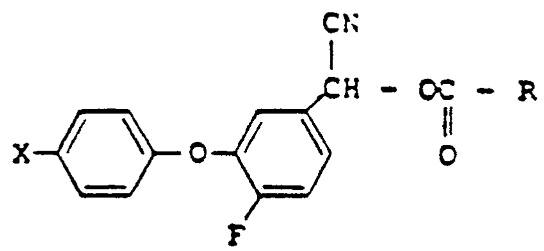

(I)

dans laquelle X a la même signification que ci-dessus ; R signifie un atome d'hydrogène, un groupe alcoyle ayant de 1 à 18 atomes de carbone, un groupe alcényle ayant de 2 à 18 atomes de carbone, un groupe alcynyle ayant de 2 à 18 atomes de carbone, un groupe alcoyle halogéné ayant de 1 à 4 atomes de carbone, un groupe alcényle halogéné ayant de 2 à 4 atomes de carbone, un groupe alcynyle halogéné ayant de 2 à 4 atomes de carbone, un groupe alcoxy ayant de 1 à 8 atomes de carbone, un groupe alcényloxy ayant de 2 à 8 atomes de carbone ou un groupe alcynyloxy ayant de 2 à 8 atomes de carbone,
de manière à hydrolyser l'ester asymétriquement jusqu'à ce qu'il se dédouble en un alcool α-cyano-benzylique riche en isomère-(S), de formule (II), et en son ester antipode.

2. Procédé suivant la revendication 1, dans lequel l'estérase provient d'un microorganisme appartenant au genre Arthrobacter, au genre Alcaligenes, au genre Achromobacter, au genre Pseudomodonas ou au genre Chromobacterium.

3. Procédé suivant la revendication 1 ou 2, dans lequel le groupe substituant R de l'ester de formule (I) est un groupe alcoyle ayant de 1 à 11 atomes de carbone, un groupe alcényle ayant de 2 à 11 atomes de carbone, un groupe alcynyle ayant de 2 à 11 atomes de carbone, un groupe alcoyle ayant de 1 à 4 atomes de carbone et ayant un atome de chlore ou un atome de brome en sa position-α, un groupe alcényle ayant de 2 à 4 atomes de carbone et ayant un atome de chlore ou un atome de brome en sa position-α, un groupe alcynyle ayant de 2 à 4 atomes de carbone et ayant un atome de chlore ou un atome de brome en sa position-α, ou un groupe alcoxy ayant de 1 à 3 atomes de carbone.

4. Procédé suivant la revendication 1, 2 ou 3, dans lequel le groupe substituant R de l'ester de formule (I) est un groupe méthyle, un groupe éthyle, un groupe monochlorométhyle, un groupe monobromométhyle, un groupe méthoxy, un groupe éthoxy ou un groupe propoxy.

5. Procédé suivant l'une quelconque des revendications 1 à 4, qui consiste à effectuer la réaction d'hydrolyse asymétrique à un pH de 3,5 à 6,0.

6. Procédé suivant l'une quelconque des revendications 1 à 5, qui consiste à effectuer la réaction d'hydrolyse asymétrique entre 20 et 50° C.

**Patentansprüche**

1. Ein Verfahren zur Herstellung einer optisch aktiven α-Cyano-benzylalkoholverbindung, die reich an (S)-Isomeren ist, dargestellt durch die Formel (II):

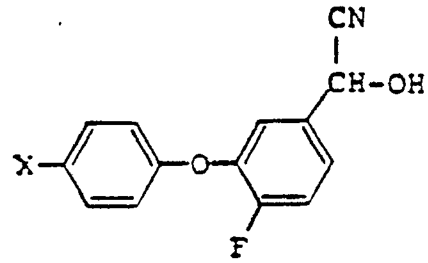

(II)

in welcher X ein Wasserstoffatom, ein Fluoratom, Chloratom oder Bromatom ist, in welchem eine aus einem Mikroorganismus, der einer Gattung angehört, die aus Arthrobacter, Alcaligenes, Achromobacter, Pseudomonas, Chromobacterium, Bacillus, Nocardia, Rhodotorula, Candida, Rhizopus, Mucor, Aspergillus, Hansenula und Torulopsis ausgewählt ist, stammende Esterase bei einem pH-Wert von 7 oder weniger mit einem Ester des (R,S)-Isomers der α-Cyano-benzylalkoholverbindung umgesetzt wird,wobei der Ester dargestellt ist durch die Formel

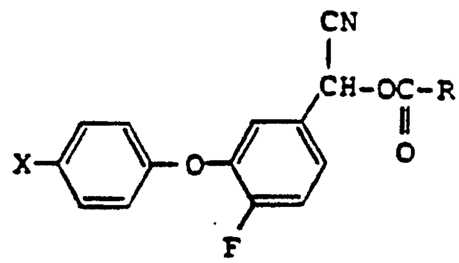

(I)

in welcher X die gleiche Bedeutung hat, wie oben erwähnt; R ein Wasserstoffatom, eine $C_1$-$C_{18}$-Alkyl-, eine $C_2$-$C_{18}$-Alkenyl-, eine $C_2$-$C_{18}$-Alkynyl-, eine halogen-substituierte $C_1$-$C_4$-Alkyl-, eine halogensubstituierte $C_2$-$C_4$-Alkenyl, eine halogen-substituierte $C_2$-$C_4$Alkynyl-,eine $C_1$-$C_8$-Alkoxy-, $C_2$-$C_8$- Alkenyloxy- oder $C_2$-$C_8$-Alkynyloxygruppe ist, um den Ester asymmetrisch zu hydrolysieren, bis er in eine α-Cyano-benzylalkoholverbindung , die reich an (S)-Isomeren ist, die durch die Formel(II) dargestellt ist, und in einen Antipodenester davon aufgetrennt ist.

2. Ein Verfahren nach Anspruch 1, in welchem die Esterase aus einem Mikroorganismus stammt, der der Gattung Arthrobacter, Alcaligenes, Achromobacter, Pseudomonas oder Chromobacterium angehört.

3. Ein Verfahren nach Anspruch 1 oder 2, in welchem die substituierende Gruppe R in dem Ester der Formel(I) eine $C_1$-$C_{11}$-Alkylgruppe, eine $C_2$-$C_{11}$-Alkenylgruppe, $C_2$-$C_{11}$-Alkynylgruppe, eine $C_1$-$C_4$-Alkylgruppe mit einem Chloratom oder einem Bromatom in deren α-Stellung, eine $C_2$-$C_4$-Alkenylgruppe mit einem Chloratom oder Bromatom in deren α-Stellung, eine $C_2$-$C_4$-Alkynylgruppe mit einem Chloratom oder einem Bromatom in deren α-Stellung, oder eine $C_1$-$C_3$-Alkoxygruppe ist.

4. Ein Verfahren nach Anspruch 1, 2 oder 3, in welchem die substituierende Gruppe R in dem Ester der Formel(I) eine Methyl-, Äthyl-, Monochlormethyl-, Monobrommethyl-, Methoxy-, Äthyoxy- oder Propoxygruppe ist.

5. Ein Verfahren nach einem der Ansprüche 1 bis 4, in welchem die asymmetrische Hydrolysereaktion bei einem pH-Wert von 3,5 bis 6,0 durchgeführt wird.

6. Ein Verfahren nach einem der Ansprüche 1 bis 5, in welchem die asymmetrische Hydrolysereaktion bei 20 bis 50° C durchgeführt wird.